# EUROPÄISCHE PATENTSCHRIFT

(11) **EP 0 494 476 B1**
(45) Veröffentlichungstag und Bekanntmachung des Hinweises auf die Patenterteilung: **14.02.1996**
(21) Anmeldenummer: 91250327.3
(22) Anmeldetag: 06.12.1991
(51) Int. Cl.: A61F 2/36, A61F 2/30

(54) **Schaftprothese**
Shaft prosthesis
Tige pour prothèse

(30) Priorität: 12.12.1990 DE 4040106
(43) Veröffentlichungstag der Anmeldung: 15.07.1992
(73) Patentinhaber: Johnson & Johnson Professional Products GmbH, D-22848 Norderstedt (DE)
(72) Erfinder: Kranz, Curt, Dr., W-1000 Berlin 62 (DE)
(74) Vertreter: UEXKÜLL & STOLBERG

(56) Entgegenhaltungen:
- EP-A- 0 190 446
- EP-A- 0 289 922
- EP-A- 0 373 011
- EP-A- 0 375 599
- DE-A- 2 933 237

## Beschreibung

Die Erfindung betrifft eine Schaftprothese der im Oberbegriff des Anspruchs 1 angegebenen Art.

Der Schaft einer derartigen Endoprothes wird bei der Implantation in einen entsprechend vorgeraspelten Knochenhohlraum fixiert, insbesondere einzementiert oder für zementfreie Implantation zum gelenkfernen Schaftende hin konisch oder auch keilartig ausgeführt, und in den entsprechend angepaßt vorbereiteten Knocheninnenraum eingeschlagen, so daß sie durch einen sogenannten Preßsitz mit dem Knochen verbunden sind.

Aus der DE-A-29 33 237 ist ein Prothesenschaft bekannt, der bezüglich seiner lokalen Elastizität und Steifigkeit an die umgebenden Bereiche des Knochens angepaßt ist. Diese Anpassung bezieht sich aber lediglich auf die Querschnittsabmessungen und ihre Abstimmung auf in Richtung der Längsachse wirkenden Kräfte. Querschnitt und Wandstärke nehmen vom oberen zum unteren Ende des Prothesenschaftes kontinuierlich ab.

Nachteilig ist dabei, daß die bei außerhalb der Längsachse angreifenden Kräften auftretenden Verformungen sich nicht konform zu den entsprechenden Verformungen des angrenzenden Knochenbereichs verhalten. Dadurch sind in Folge Mikrobewegungen und lokale Lockerungen der Prothese möglich.

Aus der EP-A-0 375 599 ist eine Endoprothese mit den Merkmalen des Oberbegriffs des Patentanspruchs 1 vorbekannt. Sie ist mit einem in den Knochenhohlraum implantierbaren, gebogenen und sich bezüglich Durchmesser und Materialquerschnitt verjüngenden Schaft derart versehen, daß die Biegesteifigkeit des Schaftes vom gelenknahen zum gelenkfernen Ende hin relativ stärker abnimmt als die Längssteifigkeit. Dies wird entsprechend der vorgeschlagenen Lösung dadurch erreicht, daß zur Verringerung der Biegesteifigkeit mindestens ein sich bis zum gelenkfernen Ende hin erstreckender Bereich ausgespart ist, der an der Innenseite der Krümmung des Prothesenschaftes gelegen ist.

Bei der erfindungsgemäßen Lösung ist in vorteilhafter Weiterbildung für den Schaft eine zum gelenkfernen Ende hin sich verjüngende Hohlrohrform mit gleichmäßig abnehmender Wandstärke gewählt, wobei dem im Bereich der maximalen Krümmung in der Lateral-Medial-Ebene außen liegenden, lateralen Rohrbereich ein am gelenkfernen Ende gelegener medialer Bereich mit geeignet geringer Wandstärke gegenüberliegt. Im Bereich des gelenkfernen Endes des Schaftes, das erfindungsgemäß durch den plattenförmigen Bereich mit extrem geringer Wandstärke . abgedeckt ist, nimmt die Biegesteifigkeit, in die das Flächenträgheitsmoment eingeht, stark ab, während die vom Materialquerschnitt bestimmte Längssteifigkeit nur geringfügig abnimmt.

Durch die trotz erfindungsgemäß geschlossener Bauform erreichte lokale Anpassung der Biegesteifigkeit des Prothesenschaftes an die des umgebenden Knochens wird das Prinzip beibehalten, daß die Längssteifigkeit des Schaftes entsprechend dem Prinzip der angepaßten Verformung eine zum gelenkfernen Ende hin stark zunehmende Fähigkeit aufweist, der Biegung des Röhrenknochens unter Belastung zu folgen.

Die Wahl einer geschlossenen Rohrform am gelenkfernen Ende des Schaftes sichert neben dem gewünschten günstigen Biege- und Längssteifigkeitsverlaufs, daß es zu keinem, grundsätzlich irreversiblen, Hineinwachsen von Knochenmaterial in den Hohlraum des Schaftes kommen kann, was sonst zu erheblichen Schwierigkeiten bei einer möglicherweise durchzuführenden Reimplantation und der dabei durchzuführenden Entfernung des bisherigen Prothesenschafts führen würde.

Die vorgenannte Lösung weist den Nachteil auf, daß der Innenraum des Schafts von unten her zugänglich und somit unerwünschten Einwirkungen während der Implantationszeit ausgesetzt ist.

Der Erfindung liegt damit die Aufgabe zugrunde, bei einer Schaftprothese der eingangs genannten Gattung die durch die konstruktive Gestaltung des Prothesenschaftes erzielte Anpassung der Verformbarkeit an die entsprechenden Eigenschaften der kortikalen Röhre des Knochens in ihren positiven Auswirkungen für den Zeitraum der Implantation dadurch sicherzustellen und zu bewahren, daß das Eindringen von Knochengewebe in den Schaftinnenraum verhindert ist.

Diese Aufgabe wird mit den kennzeichnenden Merkmalen des Anspruchs 1 gelöst.

Die Erfindung geht von der Erkenntnis aus, daß die bisher üblichen Endoprothesen, mit Ausnahme der isoelastischen, eine im Vergleich zum kortikalen Knochenrohr wesentlich höhere Biege- und Längssteifigkeit besitzen und damit eine Entlastung der Kortikalis bewirken. Der dadurch gleichzeitig entstehende Steifigkeitssprung am gelenkfernen Schaftende führt bei der implantierten Prothese zu einer Unstetigkeit der Spannungsverteilung. Der sprunghafte Anstieg der Längsspannung bewirkt eine verstärkte Kallusbildung infolge einer Kortikalisreizung. Die daraus resultierende Sockelbildung bildet jedoch ein Hindernis für ein an sich erwünschtes geringes postoperatives Einsinken und die danach notwendige Neuverankerung des Prothesenschaftes. Außerdem nimmt dadurch die Abweichung von den physiologisch normalen Verhältnissen immer mehr zu.

Durch die gattungsgemäße Prothese wurde dagegen - bei gleichzeitig ausreichender Biege- und Längssteifigkeit im gelenknahen Bereich - ein Spannungssprung durch eine verringerte Biegesteifigkeit im gelenkfernen Bereich verhindert. Hierbei wird durch eine - insbesondere nichtlinear - abnehmende Längssteifigkeit vom gelenknahen zum gelenkfernen Ende des Schaftes eine gleichmäßige und physiologisch günstige Krafteinleitung in den Knochen erreicht. Die Biegesteifigkeit nimmt vom gelenknahen zum gelenkfernen Schaftende zunächst stark ab, um im gelenkfernen Teil (etwa das restliche Drittel der Schaftlänge) nur noch langsam abzusinken.

Bei der bisherigen offenen, ausschließlich auf die zu erzielende Festigkeit ausgelegten, Bauform werden die erzielten Vorteile zum Teil wieder dadurch beeinträchtigt, daß Knochensubstanz in den Protheseninnenraum einwachsen kann.

Ein Abschluß des Prothesenschaftes mit der üblichen Wandstärke würde die erwünschten Festigkeitseigenschaften wieder zunichtemachen. Es wurde nun gefunden, daß mit den erfindungsgemäßen Maßnahmen ein Verschließen des Protheseninnenraums unter Beibehaltung der gewünschten Eigenschaften durch Wahl einer entsprechend geringen Wandstärke möglich ist. Diese Wandstärke in dem genannten Bereich ist so gewählt, daß einerseits keine die gewünschten Festigkeitseigenschaften der Prothese beeinträchtigende Zunahme der Biegesteifigkeit im gelenkfernen Bereich auftritt, andererseits aber ein Eindringen von Knochenmaterial in den Protheseninnenraum beim Implantieren und während der Implantationszeit verhindert ist. Durch in den Protheseninnenraum einwachsende Knochensubstanz wird nicht nur die spätere Entfernbarkeit des Schaftes behindert (da dieser beim Stand der Technik zum gelenkfernen Ende hin offene Innenraum bei eingesetzter Prothese nicht durch Werkzeuge zum Ausräumen erreichbar ist), sondern auch die Festigkeitseigenschaften zusätzlich verändert, da das das freie Ende der Prothese von Innen und außen umgreifende Knochenmaterial die infolge seiner Elastizität wünschenwerten Eigenbewegungen des Prothesenschafts im Bereich des gelenkfernen Endes blockiert.

Insbesondere ist weiterhin im Bereich des Adamschen Bogens eine besondere Profilierung vorgesehen, wenn die erzielbare lokale Steifigkeit durch die sich über die Schaftlänge erstreckende Wandquerschnittsabnahme nicht ausreichend ist. Die Profilierung ist vorzugsweise nicht an der konvexen, in der maximalen Krümmung nach außen weisenden Seite des Schaftes anzubringen, sondern auf den Außenflächen der lateralen Halbschale des Schaftes parallel zur Ebene der maximalen Krümmung des Schaftes im Bereich des Adamschen Bogens.

Bei einer anderen vorteilhaften Ausgestaltung der Erfindung besteht die Profilierung auf der Schaftaußenfläche aus eingearbeiteten, runden Noppen mit kugeliger Oberfläche. Die Einarbeitung der Profilierung ist so vorgenommen, daß die höchsten Profilierungspunkte auf einer (gedachten) Linie einer unprofilierten Schaftoberfläche liegen.

Die Wahl der Profiltiefe erfolgt insbesondere derart, daß einerseits der gewünschte Steifigkeitsverlauf des Hohlschaftes erreicht und andererseits verhindert wird, daß sich ein irreversibler Verbund zwischen Schaft und Knochenmaterial bilden kann. Ein solcher irreversibler Verbund würde nämlich - wie erwähnt - zu erheblichen Schwierigkeiten bei einer möglicherweise notwendig werdenden Explantation der Schaftprothese führen.

Bei einer anderen günstigen Variante der Erfindung wurde zwischen Wandstärke des Schaftes und Profiltiefe auf der Schaftoberfläche ein Verhältnis von bis zu eins zu vier als besonders vorteilhaft ermittelt. Ein entsprechendes Verhältnis wird auch für die Höhe und maximalen Durchmesser der einzelnen Noppen gewählt.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein Ausführungsbeispiel der erfindungsgemäßen Schaftprothese mit Steckkonus in Seitenansicht,
Figur 2 einen Querschnitt durch die Schaftprothese längs der Linie II...II in Figur 1,
Figur 3 einen Längsschnitt durch die Schaftprothese nach Figur 1 in der Lateral-Medial-Ebene,
Figur 4 einen Querschnitt durch die Schaftprothese längs der Linie IV...IV in Figur 3.

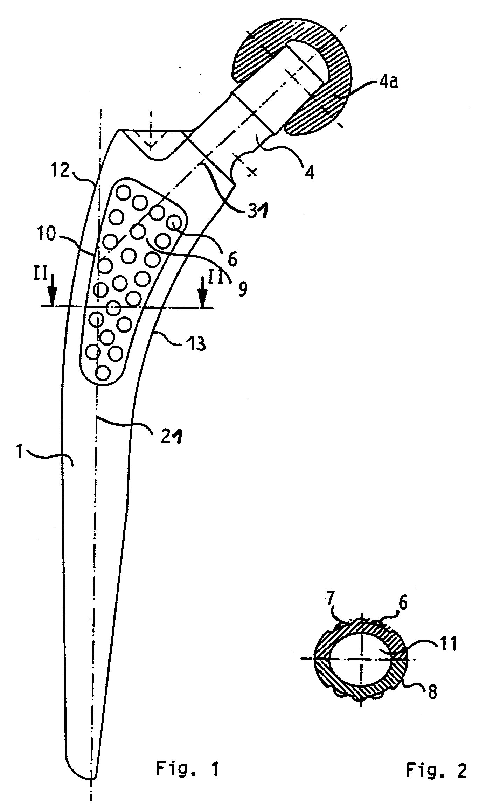

Der Schaft 1 eines in Figur 1 in der Außenansicht dargestellten Ausführungsbeispiels der erfindungsgemäßen Schaftprothese in der Ausführung als Hüftgelenkprothese besteht aus zwei gepreßten oder geschmiedeten und dann miteinander verschweißten,aus einer körperverträglichen Titanlegierung bestehenden Halbschalen, welche in der Zeichnung nicht näher dargestellt sind. Dabei bildet eine Halbschale den inneren, medialen Teil und die andere Halbschale den äußeren, lateralen Teil des Schaftes 1. Die Trennlinie verläuft dabei in etwa entlang einer gebogenen Linie welche sich in ihren Endbereichen den strichpunktierten Mittellinien 21 und 31 annähert. Die äußere Form des Schaftes 1 entspricht etwa der eines leicht gebogenen konischen Rohres und ist somit der natürlichen Form des ausgeräumten Markraumes des Femurs angepaßt. Der Prothesenhals 4 trägt an seinem Konusende eine Gelenkkugel 4a.

Ein im Bereich des Adamschen Bogens vorgesehener profilierter Oberflächenbereich 9 ist durch die Linie 10 begrenzt. Der Abstand dieser Linie zu der lateralen Hüllkurve 12 bzw. der medialen Hüllkurve 13 des Prothesenhohlschaftes 1 entspricht dabei der jeweiligen Wandstärke des Schaftes 1.

In Figur 2 ist ein Querschnitt durch den Schaft der Prothese längs der Linie II...II in Figur 1 dargestellt. Hierin ist insbesondere die erfindungsgemäße Form und Anordnung der noppenartigen Profilierung des Prothesenschaftes im Bereich des Adamschen Bogens erkennbar. Die höchsten Punkte 6 der Noppen 7 liegen dabei (bei Betrachtung im Querschnitt) auf einer gedachten Linie, die der verlängerten Hüllkurve 8 einer unprofilierten Schaftoberfläche entspricht. Die profilierte Fläche 9 entpricht in ihrer auf eine Querschnittsebene projizierten Ausdehnung im wesentlichen der Querschnittsfläche des vom Schaft eingeschlossenen Hohlraums 11. Es hat sich bezüglich der geforderten mechanischen Eigenschaften des Schaftes als vorteilhaft herausgestellt, für das Verhältnis von Profiltiefe zu Materialstärke der Schaftwandung bzw. Noppenhöhe zu maximalen Noppendurchmesser einen Wert von etwa eins zu vier zu wählen.

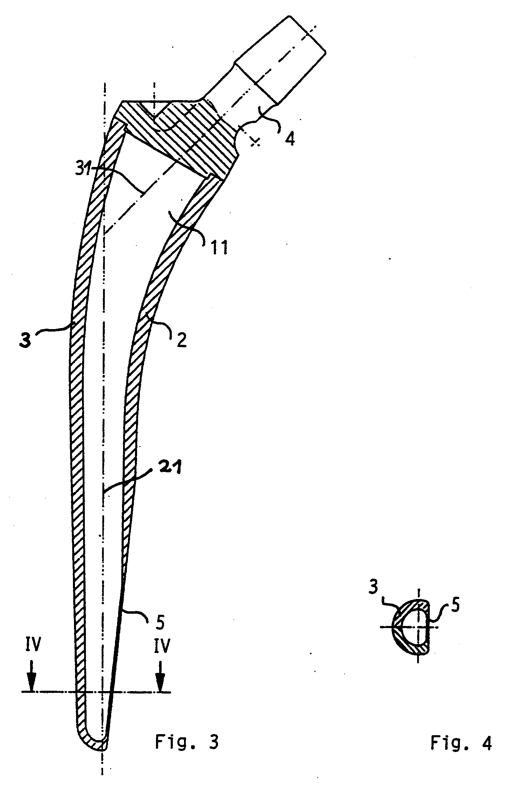

In Figur 3 ist das Ausführungsbeispiel der erfindungsgemäßen Schaftprothese in einem in der Lateral-Medial-Ebene verlaufenden Längsschnitt dargestellt. Dabei ist in einer zur Verminderung der Biegsteifigkeit materialgeschwächten Zone zur Abdeckung des hohlen Schaftinnenraums ein im wesentlichen plattenförmiger Bereich 5 vorgesehen ist, dessen Wandstärke derart gering bemessen ist, daß trotz hermetischen Abschlusses des Innenraums die lokalen Biegesteifigkeit nicht wesentlich heraufgesetzt ist, wobei die Wandstärke des plattenförmigen Bereichs im wesentlichen 1 mm beträgt.

Durch die Ausbildung des gelenkfernen Bereichs der Halbschale 2 in Form des plattenförmigen Bereichs 5 erhält der Schaft im gelenkfernen Bereich die Form eines geschlossenen Halbrohrs. Die Wandstärke des plattenförmigen Bereichs 5 ist im Verhältnis zur übrigen Materialstärke der Schaftwandung extrem gering gewählt und ist im gesamten Bereich konstant.

Es ist aus der zeichnerischen Darstellung ersichtlich, wie die Materialstärke der Schaftwandung von der Gelenkseite hin zum gelenkfernen Ende des Schaftes kontinuierlich sowohl bei der Halbschale 2 (medialer Schaftteil) als auch beim der Halbschale 3 (lateraler Schaftteil) abnimmt. Der plattenförmige Bereich 5 ist in Richtung gelenkfernem Schaftende stärker zur Mittellinie des Schaftes geneigt als der ihm gegenüberliegende Bereich der lateralen Halbschale 3. Durch diese konstruktiven Maßnahmen wird der geforderte Längs- und Biegesteifigkeitsverlauf längs des Schaftes gesichert.

Der plattenförmige Bereich 5 am gelenkfernen Ende des Schaftes 1 entspricht in seiner Längserstreckung etwa einem Drittel der Gesamtlänge des hohlen Schaftes bis zu dem das gelenknahe Ende des Schaftes verschließenden Halsteil 4.

Es ist aus der Schnittdarstellung gemäß Figur 3 ersichtlich, daß sich der Bereich der geringen Wandstärke in der Verlängerung der Innenseite 13 des Bereichs maximaler Krümmung des Hohlschaftes zum gelenkfernen Ende hin befindet. Der plattenförmige Bereich weist dabei eine konstante wandstärke auf, wobei die Wandstärke des Schaftes in dem in Längsrichtung benachbarten Abschnitt über eine Länge, die im wesentlichen der Hälfte der Längserstreckung des plattenförmigen Bereichs entspricht, kontinuierlich abnimmt.

Der Schaftquerschnitt verjüngt sich zum gelenkfernen Ende hin, in einem vor dem plattenförmigen Bereich mit geringer Wandstärke gelegenen Abschnitt, stärker als in dem daran anschließenden Abschnitt, der den plattenförmigen Bereich einschließt.

Der plattenförmige Bereich 5 mit geringer Wandstärke ist bezüglich der Mittelachse des Schaftes zum Schaftende hin stärker geneigt als der ihm gegenüberliegende,jenseits der Mittelachse des Schafts gelegene Wandungsbereich.

Die Querschnittsform des mit dem plattenförmigen Bereich 5 verschlossenen gelenkfernen Ende des Schaftes 3 der Prothese ist in Figur 4 als Querschnitt längs der Linie IV...IV gemäß Figur 1 dargestellt, aus dem ersichtlich ist, daß der gelenkferne Endbereich des Schaftes im wesentlichen die Form eines geschlossenen Halbrohres aufweist.

Die mit den erfindungsgemäßen Maßnahmen erzeugte geschlossene Oberfläche des Schaftes 1 der Prothese verhindert ein irreversibles Einwachsen von Knochenmaterial in den Hohlraum des Schaftes und ermöglicht so problemlos eine später gegebenenfalls erforderliche Explantation der Prothese.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausfuhrungen im Rahmen der Patentansprüche Gebrauch macht.

## Patentansprüche

1. Schaftprothese, insbesondere Hüftgelenkprothese, mit einem rohrförmigen, gebogenen und sich bezüglich Durchmesser und Materialquerschnitt verjüngenden Hohlschaft (1), dessen Biegesteifigkeit vom gelenknahen zum gelenkfernen Ende hin relativ stärker abnimmt als seine Längssteifigkeit, dadurch gekennzeichnet, daß in einer zur Verminderung der Biegesteifigkeit materialgeschwächten Zone, welche in Verlängerung der Innenseite (13) des Bereichs maximaler Krümmung des Hohlschaftes zum gelenkfernen Ende hin angeordnet ist, zur Abdeckung des hohlen Schaftinnenraums ein plattenförmiger Bereich (5) vorgesehen ist, dessen Wandstärke im Verhältnis zur Wandstärke der übrigen Schaftwandung derart vermindert und gering bemessen ist, daß trotz hermetischen Abschlusses des Innenraums die lokale Biegesteifigkeit nicht wesentlich heraufgesetzt ist, wobei die Wandstärke des plattenförmigen Bereichs (5) zwischen 0,5 und 1,5 mm liegt.

2. Schaftprothese nach Anspruch 1, dadurch gekennzeichnet, daß der plattenförmige Bereich (5) eine im wesentlichen konstante Wandstärke aufweist und die Wandstärke des Schaftes in dem in Längsrichtung benachbarten Abschnitt über eine Länge, die im wesentlichen der Hälfte der Längserstreckung des plattenförmigen Bereichs entspricht, kontinuierlich abnimmt.

3. Schaftprothese nach einem der vorangegangenen Ansprüche, **dadurch gekennzeichnet,** daß der Schaftquerschnitt sich zum gelenkfernen Ende hin, in einem vor dem plattenförmigen Bereich (5) mit geringer Wandstärke gelegenen Abschnitt, stärker verjüngt als in dem daran anschließenden Abschnitt, der den plattenförmigen Bereich einschließt.

4. Schaftprothese nach Anspruch 3, **dadurch gekennzeichnet,** daß der plattenförmige Bereich (5) geringer Wandstärke bezüglich der Mittelachse des Schaftes zum Schaftende hin stärker geneigt ist als der ihm gegenüberliegende jenseits der Mittelachse des Schafts gelegene Wandungsbereich.

5. Schaftprothese nach einem der vorangegangenen Ansprüche, **dadurch gekennzeichnet,** daß sich der plattenförmige bereich (5) am gelenkfernen Ende des Schaftes vorzugsweise über im wesentlichen ein Drittel der Gesamtlänge des Schaftes erstreckt.

6. Schaftprothese nach einem der vorangegangenen Ansprüche, **dadurch gekennzeichnet,** daß der gelenkferne Endbereich des Schaftes (1) im wesentlichen die Form eines geschlossenen Halbrohres aufweist.

7. Schaftprothese nach einem der vorangegangenen Ansprüche, **dadurch gekennzeichnet,** daß auf der Außenoberfläche der Wandung des Hohlschaftes eine Profilierung vorgesehen ist.

8. Schaftprothese nach Anspruch 7, **dadurch gekennzeichnet,** daß die Profilierung - vorzugsweise beidseitig jeweils - in einem Bereich der Schaftoberfläche vorgesehen ist, welche sich im wesentlichen parallel zur Ebene der maximalen Krümmung des Schaftes im Bereich des Adamschen Bogens erstreckt.

9. Hohlschaftprothese nach Anspruch 7, **dadurch gekennzeichnet,** daß die Profilierung aus verrundeten Noppen (6, 7) mit sphärischer Oberfläche besteht.

10. Hohlschaftprothese nach Anspruch 7, **dadurch gekennzeichnet,** daß die Profilierung derart in die Schaftoberfläche eingearbeitet ist, daß die höchsten Erhebungen des Profils nicht über die sich in Fortsetzung der unprofilierten Außenoberfläche des Schafts erstreckende Oberflächenkontur hinausragen, wobei die Profiltiefe und die jeweilige Materialstärke des Hohlschaftes vorzugsweise in einem Verhältnis von im wesentlichen weniger als eins zu vier stehen.

11. Hohlschaftprothese nach einem der vorangehenden Ansprüche, **dadurch gekennzeichnet,** daß der Schaft aus zwei Halbschalen zusammengefügt ist.

## Claims

1. A shaft prosthesis, in particular a hip joint prosthesis, comprising a tubular, curved hollow shaft which is tapered in diameter and in thickness and whose flexural rigidity decreases from its proximal end to its distal end at a relatively greater rate than its longitudinal rigidity, characterized in that in a material reduced zone provided for reducing the flexural rigidity, which zone is located in prolongation of the inner side (13) of the portion of the hollow shaft having the maximum curvature towards the distal end, a plate-shaped region (5) is provided for closing the cavity of the hollow shaft, the wall thickness of which region is reduced in comparison to the remaining shaft walls in such a manner that in spite of hermetically sealing the cavity the local flexural rigidity is not substantially increased, the wall thickness of the plate-shaped region (5) being between 0,5 and 1.5 mm.

2. A shaft prosthesis according to claim 1, characterized in that said plate-shaped portion (5) has a substantially uniform wall thickness, and that the wall thickness of the shaft in the longitudinally adjacent portion is continuously decreasing over a length corresponding essentially to one half of the lengthwise extension of the plate-shaped region.

3. A shaft prosthesis according to any of the preceedings claims, characterized in that the cross-sectional area of a portion ahead the plate-shaped region (5) of reduced wall thickness is tapered towards said distal end at a greater rate than in the portion subsequent thereto which includes the plate-shaped region.

4. A shaft prosthesis according to claim 3, characterized in that the plate-shaped region (5) of reduced wall thickness is steeper inclined with respect to the middle axis of said shaft towards the end of the shaft than the opposing wall portion being located beyond the middle axis of the shaft.

5. A shaft prosthesis according to any of the preceeding claims, characterized in that the plate-shaped region (5) at the distal end of the shaft extends preferrably essentially over one third of the total length of the shaft.

6. A shaft prosthesis according to any of the preceeding claims, characterized in that the distal end portion of the shaft (1) has essentially the shape of a closed half tube.

7. A shaft prosthesis according to any of the preceeding claims, characterized in that on the outer surface of the walls of the hollow shaft a profile is provided.

8. A shaft prosthesis according to Claim 7, characterized in that said profile is provided in a portion of the shaft surface, preferrably on both sides, which is essentially parallel to the plane of maximum curvature of the shaft in a region of shenton's arc.

9. A hollow shaft prosthesis according to claim 7, characterized in that that profile consists of rounded nubs (6, 7) having a spherical surface.

10. A hollow shaft prosthesis according to claim 7, characterized in that the profile being provided in the surface of the shaft in such a manner that the highest elevation of the profile does not extend beyond the extended envelope curve of the non-profiled outer surface, the depth of the profile and the respective wall thickness of the hollow shaft preferrably having a ratio of essentially less than 1 to 4.

11. A hollow shaft prosthesis according to any of the preceeding claims, characterized in that said shaft if formed of two half shells secured to one another.

## Revendications

1. Prothèse à tige, notamment prothèse d'articulation de hanche, conprenant une tige creuse (1) tubulaire,incurvée et qui s'amincit en diamètre et en section de matière, dont la rigidité à la flexion décroit plus fortement de l'extrémité proche de l'articulation à l'extrémité éloignée de l'articulation que sa rigidité longitudinale, caractérisée en ce que, dans une zone qui est affaiblie en matière pour réduire la rigidité à la flexion, qui est disposée dans le prolongement du côté intérieur (13) de la région de courbure maximale de la tige creuse en direction de l'extrémité éloignée de l'articulation, il est prévu pour fermer le volume intérieur creux de la tige, une région (5) en forme de plaque dont l'épaisseur de paroi est réduite, relativement à l'épaisseur de paroi du reste de la paroi de la tige, et est suffisamment faible pour qu'en dépit de la fermeture hermétique du volume intérieur, la rigidité locale à la flexion ne soit pas sensiblement augmentée, l'épaisseur de paroi de la région (5) en forme de plaque étant d'entre 0,5 mm et 1,5 mm.

2. Prothèse à tige selon la revendication 1, caractérisée en ce que la région (5) en forme de plaque présente une épaisseur de paroi sensiblement constante et l'épaisseur de paroi de la tige décroît continuellement dans le segment adjacent dans la direction longitudinale, sur une longueur qui correspond sensiblement à la moitié de la dimension longitudinale de la région en forme de plaque.

3. Prothèse à tige selon une des revendications précédentes, caractérisée en ce qu'en direction de l'extrémité éloignée de l'articulation, la section de la tige s'amincit plus fortement dans un segment situé avant la région (5) en forme de plaque, de plus faible épaisseur de paroi, que dans le segment qui y est adjacent et qui renferme la région en forme de plaque.

4. Prothèse à tige selon la revendication 3, caractérisé en ce que la région (5) en forme de plaque, de plus faible épaisseur de paroi, est plus fortement inclinée par rapport à l'axe médian de la tige en direction de l'extrémité de la tige, que la région de paroi qui lui fait face et qui est située de l'autre côté de l'axe médian de la tige.

5. Prothèse à tige selon une des revendications précédentes, caractérisée en ce que la région (5) en forme de plaque à l'extrémité de la tige qui est éloignée de l'articulation s'étend de préférence sur à peu près un tiers de la longueur totale de la tige.

6. Prothèse à tige selon une des revendications précédentes, caractérisée en ce que la région d'extrémité de la tige (1) qui est éloignée de l'articulation présente sensiblement la forme d'un demi-tube fermé.

7. Prothèse à tige selon une des revendications précédentes, caractérisée en ce qu'il est prévu une sculpture sur la surface extérieure de la paroi de la tige creuse.

8. Prothèse à tige selon la revendication 7, caractérisée en ce que la sculpture est prévue - de préférence des deux côtés - dans une région de la surface de la tige qui s'étend sensiblement parallèlement au plan de la courbure maximum de la tige dans la région du col du fémur.

9. Prothèse à tige creuse selon la revendication 7, caractérisée en ce que la sculpture est composée de bossettes arrondies (6, 7) à surface sphérique.

10. Prothèse à tige creuse selon la revendication 7, caractérisée en ce que la sculpture est usinée dans la surface de la tige de manière que les protubérances les plus hautes du profil ne fassent pas saillie au-dessus du contour de surface extérieure qui s'étend dans le prolongement de la surface extérieure non sculptée de la tige, la profondeur du profil et l'épaisseur de matière correspondante de la tige creuse étant de préférence liés par un rapport sensiblement inférieur à une valeur de un à quatre.

11. Prothèse à tige creuse selon une des revendications précédentes, caractérisée en ce que la tige est formée par assemblage de deux demi-coquilles.
